# EUROPEAN PATENT APPLICATION

(11) **EP 4 431 492 A1**
(43) Date of publication of application: **18.09.2024**
(21) Application number: 22892634.1
(22) Date of filing: 28.10.2022
(51) Int. Cl.: C07C 405/00, A61K 8/30, A61K 31/5575, A61P 17/14, A61P 27/02, A61Q 7/00, C07C 69/734, C07D 307/935

(54) **COMPOUND, COMPOSITION, METHOD, AND PRODUCTION METHOD**

(30) Priority: 11.11.2021 JP 2021184365
(71) Applicant: Warrantee Inc., Osaka-shi, Osaka 541-0045 (JP)
(72) Inventor: SHONO Yusuke, Osaka-shi, Osaka 541-0045 (JP); MAITI Bhimcharan, Hyderabad-500076 (IN)
(74) Representative: Hoffmann Eitle
(86) International application number: PCT/JP2022/040556
(87) International publication number: WO 2023/085135

(57) **Abstract**

At least one object of the present invention to provide a novel compound.

A compound represented by Chemical Formula (i): 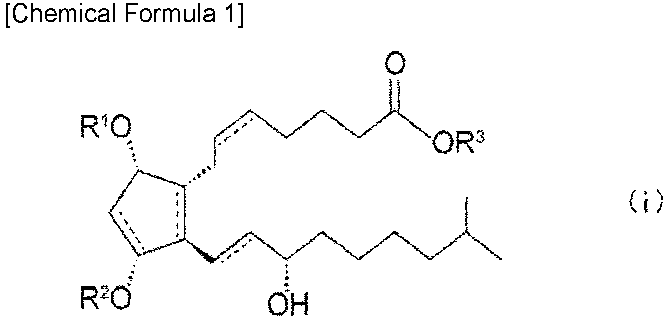 wherein a bond indicated by parallel broken and solid lines represents a single bond or a double bond capable of taking a cis configuration or a trans configuration, R¹ and R² each represent hydrogen, an alkyl group having 1 to 6 carbon atoms, a cycloalkyl group having 3 to 8 carbon atoms, an aryl group having 6 to 8 carbon atoms, or R⁴-C(=O)-, and R⁴ represents an alkyl group having 1 to 6 carbon atoms, R³ represents hydrogen, an alkyl group having 1 to 6 carbon atoms, a cycloalkyl group having 3 to 8 carbon atoms, or an aryl group having 6 to 8 carbon atoms.

## Description

### Technical Field

The present invention relates to a compound, a composition, a method, and a manufacturing method.

### Background Art

As compounds having an effect of promoting hair growth of mammals, bimatoprost (refer to Patent Literature 1), minoxidil, and the like are known.

### Citation List

### Patent Literature

Patent Literature 1: Japanese Patent No. 4143034

### Summary of Invention

### Technical Problem

At least one object of the present invention to provide a novel compound.

### Solution to Problem

According to the present invention, the above object can be achieved by the following:
[1] A compound represented by Chemical Formula (i): 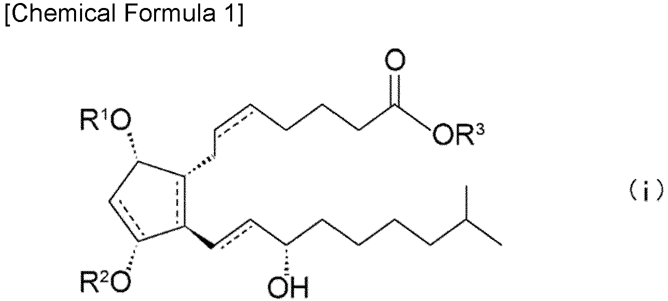 wherein a bond indicated by parallel broken and solid lines represents a single bond or a double bond capable of taking a cis configuration or a trans configuration, R¹ and R² each represent hydrogen, an alkyl group having 1 to 6 carbon atoms, a cycloalkyl group having 3 to 8 carbon atoms, an aryl group having 6 to 8 carbon atoms, or R⁴-C(=O)-, and R⁴ represents an alkyl group having 1 to 6 carbon atoms, R³ represents hydrogen, an alkyl group having 1 to 6 carbon atoms, a cycloalkyl group having 3 to 8 carbon atoms, or an aryl group having 6 to 8 carbon atoms;
[2] The compound according to [1], represented by Chemical Formula (vi): 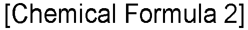 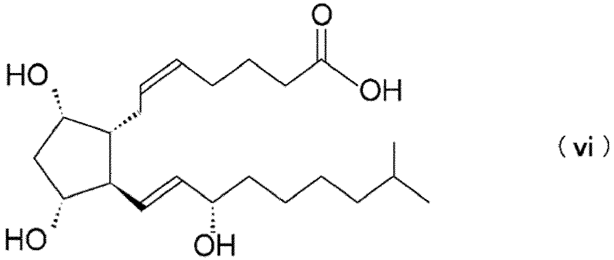 or Chemical Formula (vii): 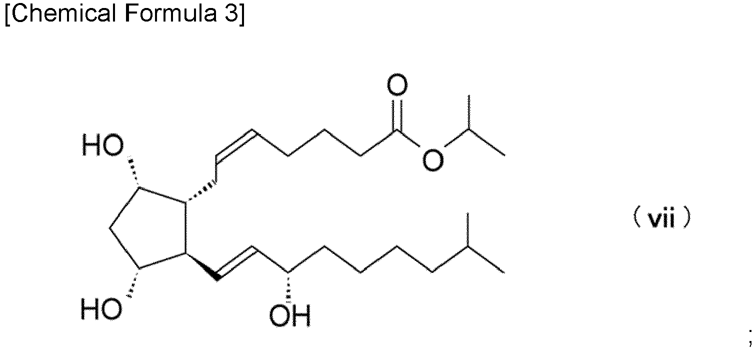
[3] The compound according to [1] or [2], wherein the compound promotes hair growth of mammals;
[4] A composition comprising the compound according to any one of [1] to [3] or a salt thereof;
[5] The composition according to [4], wherein a content of the compound or a salt thereof is 0.0005 to 5.0% by mass;
[6] The composition according to [4] or [5], comprising vitamins, wherein a content of the vitamins is 0.01 to 6.0% by mass;
[7] A method for applying the composition according to any one of [4] to [6] to mammalian skin;
[8] A manufacturing method for manufacturing a compound represented by Chemical Formula (ii): 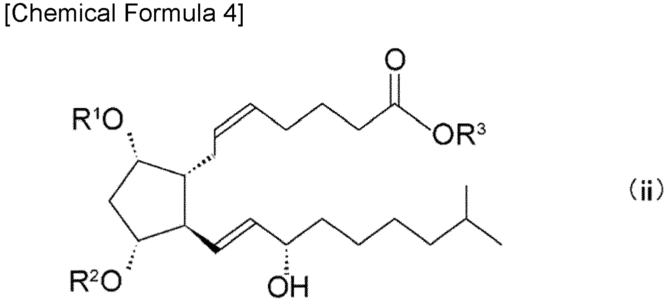 wherein R¹ and R² each represent hydrogen, an alkyl group having 1 to 6 carbon atoms, a cycloalkyl group having 3 to 8 carbon atoms, an aryl group having 6 to 8 carbon atoms, or R⁴-C(=O)-, and R⁴ represents an alkyl group having 1 to 6 carbon atoms, R³ represents hydrogen, an alkyl group having 1 to 6 carbon atoms, a cycloalkyl group having 3 to 8 carbon atoms, or an aryl group having 6 to 8 carbon atoms, the manufacturing method comprising a step of condensation of a compound represented by Chemical Formula (ix): 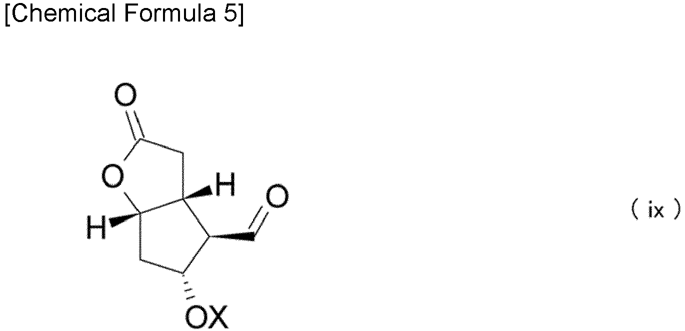 wherein X represents a hydroxyl protecting group and a compound represented by Chemical Formula (x): 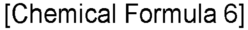 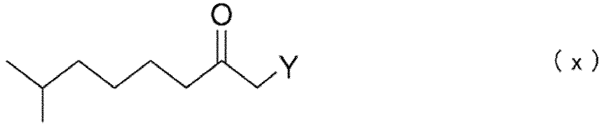 wherein Y represents a leaving group to manufacture a compound represented by Chemical Formula (xi): 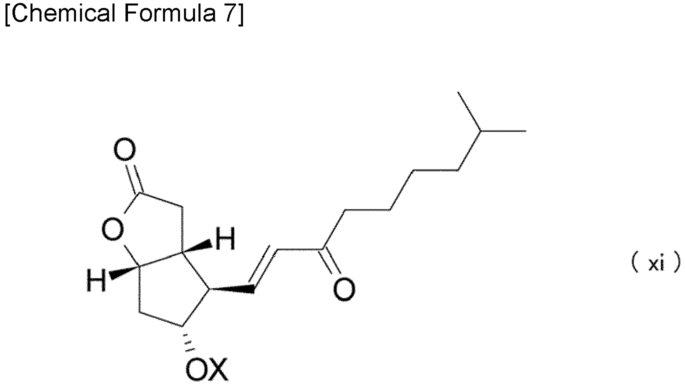 wherein X represents a hydroxyl protecting group.

### Advantageous Effects of Invention

The invention can provide a novel compound.

### Description of Embodiments

Hereinafter, embodiments of the present invention will be described, but the present invention is not limited to the following embodiments unless it is contrary to the gist of the present invention.

### (Compound)

A compound of the present invention is a compound (i) represented by the Chemical Formula (i):

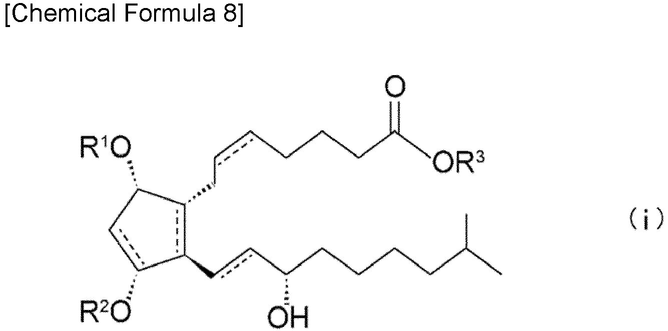

The bonds indicated by parallel broken and solid lines in Chemical Formula (i) represent a single bond or a double bond that can adopt a cis configuration or a trans configuration.

R¹ and R² each represent hydrogen, an alkyl group having 1 to 6 carbon atoms, a cycloalkyl group having 3 to 8 carbon atoms, an aryl group having 6 to 8 carbon atoms, or R⁴-C(=O)-, and R⁴ represents an alkyl group having 1 to 6 carbon atoms. R¹ and R² may be the same as or different from each other.

Further, R³ represents hydrogen, an alkyl group having 1 to 6 carbon atoms, a cycloalkyl group having 3 to 8 carbon atoms, or an aryl group having 6 to 8 carbon atoms.

The alkyl group having 1 to 6 carbon atoms adopted for R¹ to R⁴ of the compound (i) may have a linear structure, a branched structure, or an unsaturated bond. In addition, some of the hydrogen atoms in the alkyl group may be substituted with atoms other than hydrogen atoms or substituents. Examples of the alkyl group include a methyl group, an ethyl group, a n-propyl group, an isopropyl group, a n-butyl group, a t-butyl group, an isobutyl group, a sec-butyl group, a n-pentyl group, and a n-hexyl group. The alkyl groups having 1 to 6 carbon atoms adopted for R¹ to R⁴ may be the same or different.

The number of carbon atoms of the alkyl group adopted for R¹ to R⁴ is preferably 1 or more, and more preferably 2 or more. The number of carbon atoms of the alkyl group adopted for R¹ to R⁴ is preferably 6 or less, and more preferably 4 or less.

The cycloalkyl group having 3 to 8 carbon atoms adopted for R¹ to R³ of the compound (i) may be monocyclic or polycyclic as long as it is in a nonaromatic cyclic form, and may have an unsaturated bond. In addition, some of the hydrogen atoms in the cycloalkyl group may be substituted with atoms other than hydrogen atoms or substituents. Examples of the cycloalkyl group include a cyclopropyl group, a cyclobutyl group, a cyclopentyl group, a cyclohexyl group, a cycloheptyl group, and a cyclooctyl group. The cycloalkyl group having 3 to 8 carbon atoms adopted for R¹ to R³ may be the same or different.

The number of carbon atoms of the cycloalkyl group adopted for R¹ to R³ is preferably 3 or more, and more preferably 4 or more. The number of carbon atoms of the cycloalkyl group adopted for R¹ to R³ is preferably 8 or less, and more preferably 6 or less.

The aryl group having 6 to 8 carbon atoms adopted for R¹ to R³ of the compound (i) refers to an aromatic substituent. In addition, some of the hydrogen atoms in the aryl group may be substituted with atoms other than hydrogen atoms or substituents. Examples of the aryl group include a phenyl group, a tolyl group, and a xylyl group.

The number of carbon atoms of the aryl group adopted for R¹ to R³ is preferably 6 or more. The number of carbon atoms of the aryl group adopted for R¹ to R³ is preferably 8 or less, and more preferably 7 or less.

The compound (i) can provide a novel compound.

A compound represented by the Chemical Formula (i) may be a compound (ii) represented by the Chemical Formula (ii):

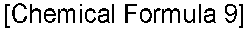

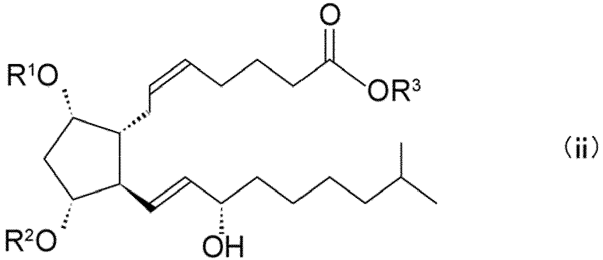

The double bond in Chemical Formula (ii) can have a cis configuration or a trans configuration. R¹ and R² each represent hydrogen, an alkyl group having 1 to 6 carbon atoms, a cycloalkyl group having 3 to 8 carbon atoms, an aryl group having 6 to 8 carbon atoms, or R⁴-C(=O)-, and R⁴ represents an alkyl group having 1 to 6 carbon atoms. R¹ and R² may be the same as or different from each other.

Further, R³ represents hydrogen, an alkyl group having 1 to 6 carbon atoms, a cycloalkyl group having 3 to 8 carbon atoms, or an aryl group having 6 to 8 carbon atoms.

With respect to the alkyl group, the cycloalkyl group, and the aryl group of the Chemical Formula (ii), the description of the compound (i) can be applied within a necessary range.

In addition, a compound represented by Chemical Formula (ii) may be a compound (iii) represented by Chemical Formula (iii):

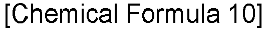

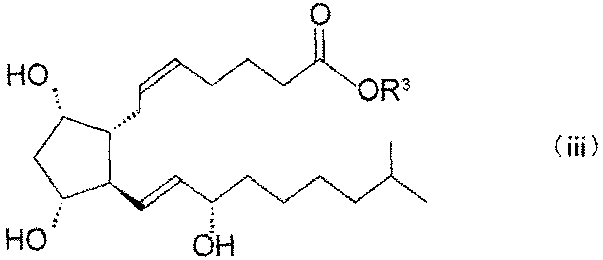

The double bond in Chemical Formula (iii) can have a cis configuration or a trans configuration. In addition, R³ represents hydrogen, an alkyl group having 1 to 6 carbon atoms, a cycloalkyl group having 3 to 8 carbon atoms, or an aryl group having 6 to 8 carbon atoms.

With respect to the alkyl group, the cycloalkyl group, and the aryl group of the Chemical Formula (iii), the description of the compound (i) can be applied within a necessary range.

In addition, a compound represented by Chemical Formula (ii) may be a compound (iv) represented by Chemical Formula (iv):

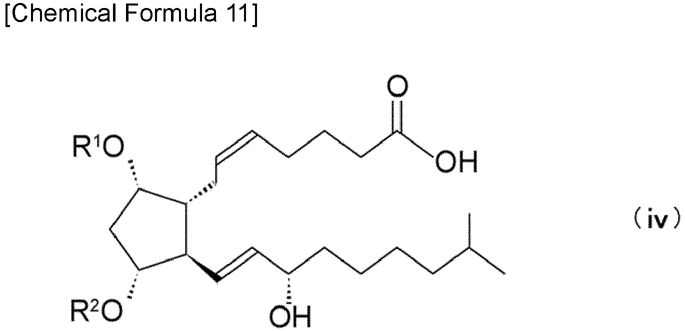

The double bond in Chemical Formula (iv) can have a cis configuration or a trans configuration. R¹ and R² each represent hydrogen, an alkyl group having 1 to 6 carbon atoms, a cycloalkyl group having 3 to 8 carbon atoms, an aryl group having 6 to 8 carbon atoms, or R⁴-C(=O)-, and R⁴ represents an alkyl group having 1 to 6 carbon atoms. R¹ and R² may be the same as or different from each other.

With respect to the alkyl group, the cycloalkyl group, and the aryl group of the Chemical Formula (iv), the description of the compound (i) can be applied within a necessary range.

In addition, a compound represented by Chemical Formula (ii) may be a compound (v) represented by Chemical Formula (v):

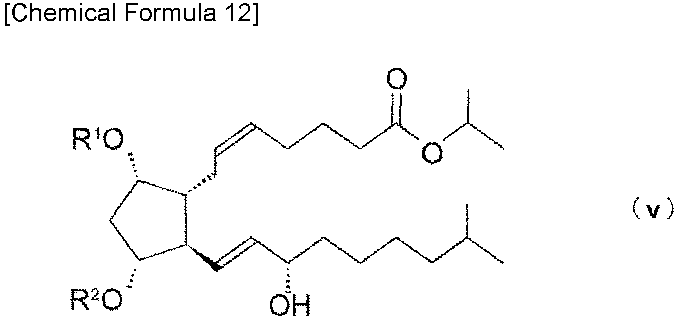

The double bond in Chemical Formula (v) can have a cis configuration or a trans configuration. R¹ and R² each represent hydrogen, an alkyl group having 1 to 6 carbon atoms, a cycloalkyl group having 3 to 8 carbon atoms, an aryl group having 6 to 8 carbon atoms, or R⁴-C(=O)-, and R⁴ represents an alkyl group having 1 to 6 carbon atoms. R¹ and R² may be the same as or different from each other.

With respect to the alkyl group, the cycloalkyl group, and the aryl group of the Chemical Formula (v), the description of the compound (i) can be applied within a necessary range.

Further, a compound of the present invention is preferably a compound (vi) represented by the Chemical Formula (vi):

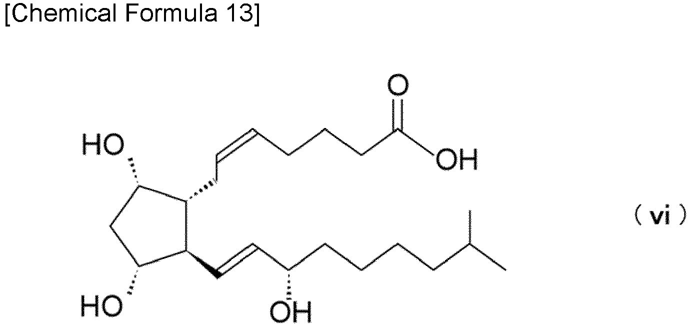

The double bond in Chemical Formula (vi) can have a cis configuration or a trans configuration.

Alternatively, a compound of the present invention is preferably a compound (vii) represented by the Chemical Formula (vii):

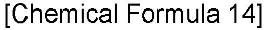

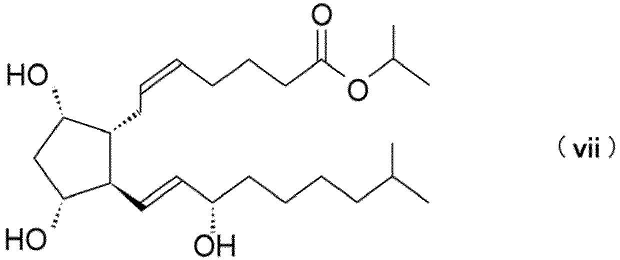

The double bond in Chemical Formula (vii) can have a cis configuration or a trans configuration.

In addition, the compound of the present invention can promote the hair growth of mammals including humans. The hair is not particularly limited as long as it is hair that grows on the epidermis of the animal body. Examples of the hair include eyelashes, head hair, eyebrows, and body hair. Hair growth refers to, for example, elongation of the currently growing hair, an increase in diameter of the currently growing hair, and development of new hair.

Hair growth may be facilitated, for example, by activation of cells and tissues related to hair. Cells and tissues related to hair include, for example, hair mother cells, hair papilla cells, and capillaries. In addition, the activation of cells and tissues related to hair includes differentiation of cells, proliferation of cells, an increase in blood flow in capillaries, and the like.

### (Composition)

The composition of the present invention contains the above-described compound or a pharmacologically acceptable salt of the compound.

The above-described compound refers to any one of the compounds (i) to (vii). The above-described compound contained in the composition of the present invention is preferably a compound represented by the Chemical Formula (i). In addition, the above-described compound contained in the composition of the present invention is more preferably a compound represented by the Chemical Formula (ii). In addition, the above-described compound contained in the composition of the present invention is still more preferably a compound represented by any one of the Chemical Formulas (iii) to (v). Further, the above-described compound contained in the composition of the present invention is particularly preferably a compound represented by the Chemical Formula (vi) or (vii).

The pharmacologically acceptable salt refers to a salt prepared from a base or acid that is acceptable for administration to mammals and the like. Examples of the pharmacologically acceptable salt include a salt generated from an acid such as a carboxyl group or a hydroxyl group of the compound of the present invention and a base such as an organic base or an inorganic base. The pharmaceutically acceptable salt can be derived from a pharmaceutically acceptable inorganic base or organic base or from a pharmaceutically acceptable inorganic acid or organic acid.

Examples of the salt derived from pharmaceutically acceptable inorganic bases include ammonium salts, calcium salts, copper salts, ferric salts, ferrous salts, lithium salts, magnesium salts, manganese salts, manganous salts, potassium salts, sodium salts, and zinc salts.

Examples of the salt derived from pharmaceutically acceptable organic bases include salts of substituted amines, cyclic amines, primary, secondary, and tertiary amines, specifically arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, methylglucamine, glucosamine, histidine, hydrabamine, isopropylamine, lysine, morpholine, piperazine, piperazine, polyamine resins, procaine, purines, theobromine, triethylamine, trimethylamine, tripropylamine, and tromethamine.

Examples of the salt derived from pharmaceutically acceptable inorganic acids include boric acid, carbonic acid, hydrohalic acid (hydrobromic acid, hydrochloric acid, hydrofluoric acid, hydroiodic acid, and the like), nitric acid, phosphoric acid, sulfamic acid, and sulfuric acid.

Examples of the salts derived from pharmaceutically acceptable organic acids include salts of aliphatic hydroxyl acids (citric acid, gluconic acid, glycolic acid, lactic acid, lactobionic acid, malic acid, tartaric acid, and the like), aliphatic monocarboxylic acids (acetic acid, butyric acid, formic acid, propionic acid, trifluoroacetic acid, and the like), amino acids (aspartic acid, glutamic acid, and the like), aromatic carboxylic acids (benzoic acid, p-chlorobenzoic acid, diphenylacetic acid, genticic acid, hippuric acid, triphenylacetic acid, and the like), aromatic hydroxyl acids (o-hydroxybenzoic acid, p-hydroxybenzoic acid, 1-hydroxynaphthalene-2-carboxylic acid, 3-hydroxynaphthalene-2-carboxylic acid, and the like), ascorbic acid, dicarboxylic acid (fumaric acid, maleic acid, oxalic acid, succinic acid, and the like), glucuronic acid, mandelic acid, mucic acid, nicotinic acid, orotic acid, pamoic acid, pantothenic acid, sulfonic acid (benzenesulfonic acid, camphorsulfonic acid, edicylic acid, ethanesulfonic acid, isethionic acid, methanesulfonic acid, naphthalenesulfonic acid, naphthalene-1,5-disulfonic acid, naphthalene-2,6-disulfonic acid, p-toluenesulfonic acid, and the like), and xinafoic acid.

The content of the above-described compound or a pharmacologically acceptable salt thereof in the composition is preferably 5.0% by mass or less, more preferably 1.0% by mass or less, still more preferably 0.5% by mass or less, particularly preferably 0.1% by mass or less, and particularly preferably 0.05% by mass or less. In addition, the content of the above-described compound or a pharmacologically acceptable salt thereof in the composition is preferably 0.0005% by mass or more, more preferably 0.01% by mass or more, still more preferably 0.015% by mass or more, particularly preferably 0.02% by mass or more, and particularly preferably 0.025% by mass or more.

The content of the above-described compound or the pharmacologically acceptable salt thereof in the composition is within the above range, thereby allowing hair growth to be promoted while suppressing the risk of causing side effects when the composition is applied to mammals.

In addition, the composition of the present invention preferably contains vitamins.

The vitamins refer to organic compounds that act as vitamins, and may be fat-soluble or water-soluble. Examples of the vitamins include vitamin A, vitamin B group (vitamin B₁, vitamin B₂, vitamin B₃, vitamin B₅, vitamin B₆, vitamin B₇, vitamin B₉, vitamin B₁₂), vitamin C, vitamin D, vitamin E, and vitamin K. Vitamins may be derivatives of the above vitamins.

In order to promote the excretion of melanin and suppress pigmentation at the site to which the composition is applied, the composition of the present invention preferably contains, as vitamins, vitamin C, vitamin B₂, vitamin B₃, vitamin B₆, and/or derivatives thereof.

The content of vitamins in the composition is preferably 6.0% by mass or less, more preferably 5.0% by mass or less, still more preferably 4.0% by mass or less, particularly preferably 3.0% by mass or less, and particularly preferably 2.0% by mass or less. In addition, the content of vitamins in the composition is preferably 0.01% by mass or more, more preferably 0.1% by mass or more, still more preferably 0.5% by mass or more, further preferably 0.8% by mass or more, and particularly preferably 1.0% by mass or more.

The content of vitamins in the composition is within the above range, thereby allowing the effect of the vitamins to be obtained while suppressing the risk of causing side effects when the composition is applied to mammals.

The composition of the present invention can contain various compounds in addition to the above-described compound or a pharmacologically acceptable salt thereof or vitamins. The composition of the present invention can contain, for example, a whitening component, a nonionic surfactant, a stabilizer, an excipient (buffering agent, solvent, pH adjusting agent, tonicity agent, and the like), an astringent, a fragrance, a softening agent, a coloring agent, a moisturizing agent, a propellant, an ultraviolet protecting agent, an antioxidant, a thickener, a preservative, and an additive generally used in other cosmetics.

Examples of the whitening component include L-cysteine, human oligopeptide (EGF), arbutin, chamomilla ET, hydroquinone, tranexamic acid, placenta extract, kojic acid, linoleic acid S, nicotinic acid amide, 4-methoxysalicylic acid potassium salt, magnolignan, and adenosine-sodium phosphate OT.

Examples of the nonionic surfactant include polyoxyethylene fatty acid ester, polyoxyethylene hydrogenated castor oil, polyoxyethylene polyoxypropylene glycol, and sucrose fatty acid ester.

Examples of the stabilizer include ethylenediaminetetraacetic acid, edetate disodium, dibutylhydroxytoluene, sodium nitrite, ascorbic acid, L-ascorbic acid stearate, sodium bisulfite, alpha thioglycerin, erythorbic acid, cysteine hydrochloride, citric acid, tocopherol acetate, potassium dichloroisocyanurate, 2,6-di-t-butyl-4-methylphenol, soybean lecithin, sodium thioglycolate, sodium thiomalate, natural vitamin E, tocopherol, ascorbyl pasthyminate, sodium pyrosulfite, butylhydroxyanisole, 1,3-butylene glycol, propyl gallate, 2-mercaptobenzimidazole, and oxyquinoline sulfate.

Examples of the buffer include sodium dihydrogen phosphate dihydrate, boric acid, borax, citric acid, and ε-aminocaproic acid. Examples of the tonicity agent include glycerol, sorbitol, mannitol, propylene glycol, sodium chloride, potassium chloride, and calcium chloride.

The compound formulated in the composition of the present invention is contained in a dermatologically acceptable amount. The dermatologically acceptable amount refers to an amount that, when the composition is applied to mammals, is less likely to cause side effects, such as undesirable toxicity and allergic reactions.

In the composition of the present invention, water, alcohol, oil, and the like can be used as the solvent. Water may be purified water, hot spring water, herb water, marine deep water, and the like. As the alcohol, for example, ethanol can be used. As the oil, for example, mineral oil, jojoba oil, olive oil, and horse oil can be used. In addition, in the composition of the present invention, a mixture of two or more of these may be used as the solvent. The solvent can be appropriately selected according to the form of the composition.

The content of the solvent in the composition is preferably 98.0% by mass or less, more preferably 93.0% by mass or less, and still more preferably 90.0% by mass or less. In addition, the content of the solvent in the composition is preferably 70.0% by mass or more, more preferably 80.0% by mass or more, and still more preferably 85.0% by mass or more.

The form of the composition of the present invention is not particularly limited as long as it can be applied to mammals. Examples of the form of the composition of the present invention include liquid, powder, gel, sol, ointment, cream, foam, lotion, film, shampoo, paste, and spray.

The method for applying the composition of the present invention to mammals is not particularly limited, and can be appropriately designed according to the site to be applied and the form of the composition. For example, when the composition is applied to promote the growth of eyelashes, a method such as instillation of the composition or application of the composition to the eyelashes can be adopted. When the composition is instilled, the composition spreads on the surface of the eyeball and adheres to the skin around the eyeball, thereby promoting the growth of eyelashes. When the composition is instilled, the form of the composition is preferably a liquid or the like. When the composition is applied to eyelashes, the form of the composition is preferably liquid or cream.

The number of times of application of the composition per day is preferably 1 or more, more preferably 2 or more. In addition, the number of times of application of the composition per day is preferably 5 or less, and more preferably 4 or less.

When the composition is applied for promoting the growth of eyelashes, the amount of the composition applied at a time is preferably 30 µl or more, more preferably 40 µl or more. When the composition is applied to promote the growth of eyelashes, the number of times of application per day is preferably 100 µl or less, more preferably 150 µl or less.

The composition of the present invention is preferably enclosed in an appropriate container depending on the form of the composition and the site to be applied. Examples of the form of the container of the composition include a bottle, a tube, an ampule, a pipette, and a fluid dispenser.

The material of the container is not particularly limited, and can be appropriately designed. Examples of the material of the container include resin and glass. As the resin, for example, polyethylene, polypropylene, polyethylene terephthalate, polyvinyl chloride, acrylic resin, polystyrene, polymethyl methacrylate, nylon, or the like can be used.

### (Manufacturing method)

The compound (ii) of the present invention is manufactured, for example, by the following Reaction Scheme 1:

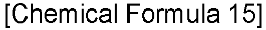

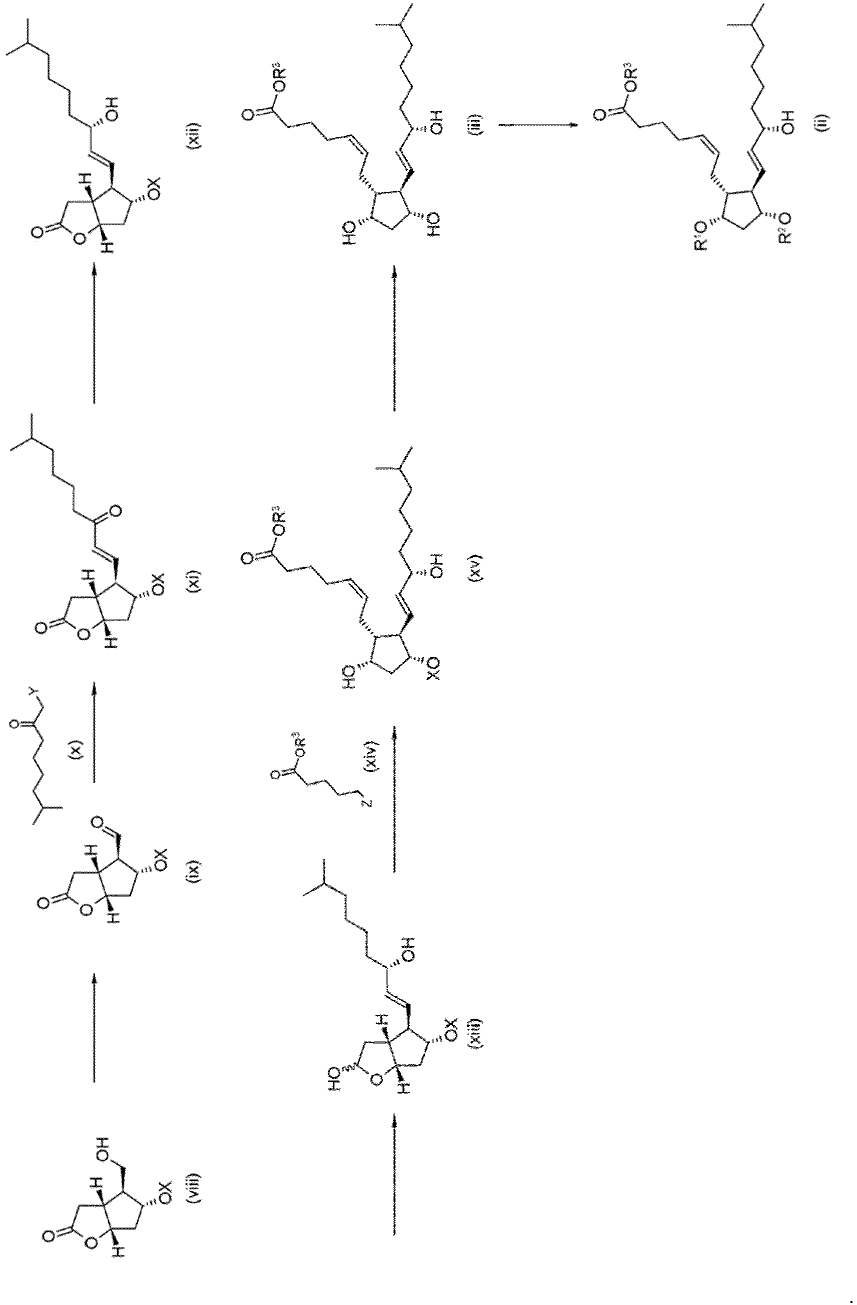

In Reaction Scheme 1, X represents a hydroxyl protecting group. The hydroxyl protecting group is not particularly limited as long as it can protect the hydroxyl group. For example, as the hydroxyl protecting group, there can be adopted a methyl group, a trityl group, a methoxymethyl group, a 1-ethoxyethyl group, a methoxyethoxymethyl group, a 2-tetrahydropyranyl group, a trimethylsilyl group, a triethylsilyl group, a tert-butyldimethylsilyl group, a tert-butyldiphenylsilyl group, an acetyl group, a pivaloyl group, a benzoyl group, a benzyl group, a p-methoxybenzyl group, an allyloxycarbonyl group, and a 2,2,2-trichloroethoxycarbonyl group. From the viewpoint of high stability, it is preferable to adopt a tert-butyldimethylsilyl group as the hydroxyl protecting group.

In Reaction Scheme 1, Y and Z represent a leaving group. The leaving group refers to a group that can be substituted with a nucleophile, and is not particularly limited as long as it can be eliminated by a condensation reaction. The leaving group may be eliminated by reacting a nucleophile in the presence of a strong base such as NaH. As the leaving group, for example, there can be adopted triphenylphosphine, dimethyl phosphonate, a halide (I, Br, F, Cl, and the like), a sulfonate (mesylate, tosylate, and the like), a sulfide (SCH₃ and the like), an ester, N-hydroxysuccinimide, and N-hydroxybenzotriazole. In addition, the nucleophile is also not particularly limited, and for example, there can be adopted an amine, a thiol, an alcohol, an anionic species (alkoxides, amides, carbanions, and the like), and a Grignard reagent. As the alkoxide, for example, potassium tert-butoxide and the like can be adopted. Y and Z may be the same as or different from each other.

In addition, in the Reaction Scheme 1, R³ represents hydrogen, an alkyl group having 1 to 6 carbon atoms, a cycloalkyl group having 3 to 8 carbon atoms, or an aryl group having 6 to 8 carbon atoms. With respect to the alkyl group, the cycloalkyl group, and the aryl group, the description of the compound (i) can be applied within a necessary range.

In the Reaction Scheme 1, first, a hydroxyl group of a compound (viii) represented by Chemical Formula (viii) is oxidized to an aldehyde group, whereby a compound (ix) represented by Chemical Formula (ix) can be obtained. The oxidation reaction is not particularly limited as long as it can oxidize a hydroxyl group to an aldehyde group and does not affect other functional groups. For example, Dess-Martin oxidation, manganese dioxide oxidation, Ley-Griffith oxidation, TEMPO oxidation, Parikh-Doering oxidation, and the like can be adopted. Dess-Martin oxidation is preferably adopted from the viewpoint of ease of operation.

Then, the aldehyde group of the compound (ix) reacts with the leaving group Y of the compound (x) represented by Chemical Formula (x) to leave hydrogen and the leaving group Y, and the compound (ix) and the compound (x) are condensed, whereby the compound (xi) represented by the Chemical Formula (xi) can be obtained. The condensation reaction is not particularly limited as long as the aldehyde group reacts with the leaving group Y and other functional groups are not affected. For example, condensation can be performed using sodium hydride, potassium tert-butoxide, or the like.

Then, the ketone group of the compound (xi) is reduced to a hydroxyl group, whereby a compound (xii) represented by Chemical Formula (xii) can be obtained. The reduction reaction is not particularly limited as long as it can reduce a ketone group to a hydroxyl group and does not affect other functional groups. For example, there can be adopted hydride reduction, metal reduction, catalytic reduction, silane reduction, tributyltin reduction, Meerwein-Ponndorf-Verley reduction, and Corey-Bakshi-Shibata reduction can be adopted. From the viewpoint of ease of operation, it is preferable to adopt Corey-Bakshi-Shibata reduction.

Then, the ester of the compound (xii) is reduced to form a hydroxyl group, whereby a compound (xiii) represented by Chemical Formula (xiii) can be obtained. The reduction reaction is not particularly limited as long as it can reduce an ester to form a hydroxyl group and does not affect other functional groups. For example, there can be adopted hydride reduction, metal reduction, catalytic reduction, silane reduction, tributyltin reduction, Meerwein-Ponndorf-Verley reduction, and Corey-Bakshi-Shibata reduction can be adopted. For the hydride reduction, for example, diisobutylaluminum hydride or the like can be used.

A compound (xv) represented by Chemical Formula (xv) can be obtained by reacting a terminal hydroxyl group generated by ring-opening an ether bond moiety of the compound (xiii) with a leaving group Z of a compound (xiv) represented by Chemical Formula (xiv), and condensing the compound (xiii) and the compound (xiv). The ring-opening reaction and the condensation reaction are not particularly limited as long as the ether bond moiety is ring-opened, the hydroxyl group and the leaving group Z react, and other functional groups are not affected. For example, sodium hydride, potassium tert-butoxide, or the like can be used.

Then, eliminating the hydroxyl protecting group of the compound (xv) can provide the compound (iii) represented by the Chemical Formula (iii). The deprotection reaction is not particularly limited as long as it is a reaction that can eliminate the hydroxyl protecting group and does not affect other functional groups. For example, there can be adopted acidic conditions, basic conditions, oxidation conditions, reduction conditions, fluoride ions, hydrogenation, and the like. When the hydroxyl protecting group is a tert-butyldimethylsilyl group, tetra-n-butylammonium fluoride can be used for the deprotection reaction.

The compound (ii) can be obtained by substituting R¹ and R² for hydrogen of the hydroxyl group bonded to the five-membered ring of the compound (iii). The substitution reaction is not particularly limited as long as the hydrogen of the hydroxyl group bonded to the five-membered ring can be substituted with R¹ and R², and the reaction does not affect other functional groups.

R¹ and R² each represent hydrogen, an alkyl group having 1 to 6 carbon atoms, a cycloalkyl group having 3 to 8 carbon atoms, an aryl group having 6 to 8 carbon atoms, or R⁴-C(=O)-, and R⁴ represents an alkyl group having 1 to 6 carbon atoms. R¹ and R² may be the same as or different from each other. With respect to the alkyl group, the cycloalkyl group, and the aryl group, the description of the compound (i) can be applied within a necessary range.

The compound (viii) as a starting material of the Reaction Scheme 1 can be manufactured, for example, by the following Reaction Scheme 2:

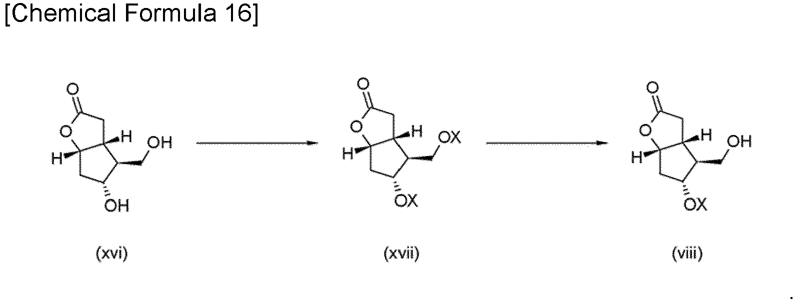

First, the compound (xvii) represented by Chemical Formula (xvii) can be obtained by protecting two hydroxyl groups of the compound (xvi) represented by the Chemical Formula (xvi) with a hydroxyl protecting group X. For example, tert-butyldimethylchlorosilane may be reacted with the compound (xvi) to introduce a tert-butyldimethylsilyl group as the hydroxyl protecting group X.

Then, in the compound (xvii), the hydroxyl protecting group X introduced into the hydroxyl group of the hydroxymethyl group is eliminated, whereby the compound (viii) can be obtained. For example, the hydroxyl protecting group X may be eliminated by acidic conditions with acetic acid.

The compound (ii) of the present invention can be manufactured by the manufacturing method including the following Reaction Scheme 3:

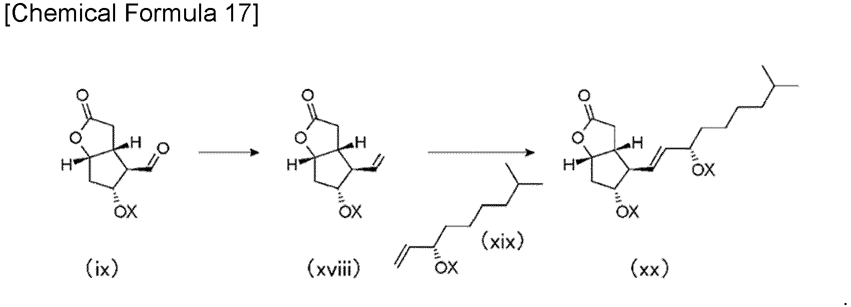

In the manufacturing method including the Reaction Scheme 3, a compound (xviii) represented by Chemical Formula (xviii) can be obtained by reducing the aldehyde group of the compound (ix) to form alkene. In order to convert the aldehyde group to alkene, there can be used Wittig reaction, Horner-Wadsworth-Emmons reaction, Peterson olefination reaction, Tebbe reagent, Petasis reagent, and Julia-Lythgoe olefination.

Then, a bond between the carbon-carbon double bond of the compound (xviii) and the carbon-carbon double bond of the compound (xix) represented by the Chemical Formula (xix) is recombined by a metathesis reaction, and a compound (xx) represented by Chemical Formula (xx) can be obtained. For the metathesis reaction, Grubbs catalyst, Shrock catalyst, or the like can be used.

The compound (iii) can be obtained by condensing the compound (xx) and the compound (xiv) to eliminate the hydroxyl protecting group. Then, the compound (ii) can be obtained by substituting R¹ and R² for hydrogen of the hydroxyl group bonded to the five-membered ring of the compound (iii). The above description can be applied to the deprotection reaction and the substitution reaction within a necessary range.

The method for manufacturing the compound (ii) according to the Reaction Scheme 1 is preferable because the manufacturing time can be shortened and a high yield can be obtained as compared with the method for manufacturing the compound (ii) including the Reaction Scheme 3.

In addition, in the Reaction Scheme 1 or 3, using another compound containing a leaving group Z and one or more carbons bonded to the leaving group Z in place of the compound (xiv) can manufacture a compound (xxi) represented, for example, by the following Chemical Formula (xxi):

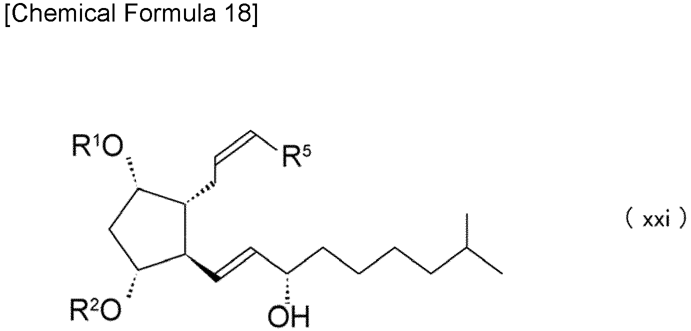

In the Chemical Formula (xxi), R⁵ is not particularly limited, and any structure can be adopted. For example, R⁵ may represent hydrogen, an alkyl group, a cycloalkyl group, an aryl group, a carboxyl group, or R⁴-C(=O)-, and R⁴ may be an alkyl group. In addition, for example, R⁵ may represent hydrogen, an alkyl group having 1 to 6 carbon atoms, a cycloalkyl group having 3 to 8 carbon atoms, an aryl group having 6 to 8 carbon atoms, a carboxyl group having 1 to 6 carbon atoms, or R⁴-C(=O)-, and R⁴ may represent an alkyl group having 1 to 6 carbon atoms.

The double bond in the Chemical Formula (xxi) can have a cis configuration or a trans configuration. R¹ and R² each represent hydrogen, an alkyl group having 1 to 6 carbon atoms, a cycloalkyl group having 3 to 8 carbon atoms, an aryl group having 6 to 8 carbon atoms, or R⁴-C(=O)-, and R⁴ represents an alkyl group having 1 to 6 carbon atoms. R¹ and R² may be the same as or different from each other.

With respect to the alkyl group, the cycloalkyl group, and the aryl group of the Chemical Formula (xxi), the description of the compound (i) can be applied within a necessary range.

As the compound used as a starting material in the Reaction Schemes 1 to 3 and the compound or reagent to be added, known compounds can be used, or manufacturing can be performed according to a known method or a method described in Examples.

### Examples

Hereinafter, the present invention will be described in more detail with reference to Examples and the like, but the present invention is not limited by these Examples at all.

### [Manufacturing of compound]

Hereinafter, unless otherwise specified, reagents and solvents were used without further purification or drying. Further, unless otherwise specified, the reactions were performed at room temperature in an inert atmosphere.

In the following Examples, analysis by NMR was performed using a 400-MHz instrument manufactured by Bruker. Software TOPSPIN 4.0.7 (manufactured by Bruker) was used. The number of scans was 32, and the relaxation delay was 1 second. In addition, a solvent CDCl₃ or DMSO-d₆ was used.

In the following Examples, analysis by the LCMS method was performed by ACQUITY UPLC (SQD-2, with ESI, manufactured by Waters Corporation). Acquity UPLC BEH C18 (2.1 mm × 50 mm, 1.7 µm) was used as a column, 0.05% formic acid aqueous solution was used as a mobile phase A, and 0.05% formic acid acetonitrile solution was used as a mobile phase B. The gradient (%B) was 0/10, 0.5/10, 1/35, 1.5/45, 2.3/90, 3.2/90, 3.6/10, and 3.8/10, the flow rate was 0.4 ml/min, the column temperature was 35°C, and the diluents were acetonitrile and water. In addition, ELSD was used as a detector.

In the following Examples, analysis by the UPLC method was performed by ACQUITY UPLC (manufactured by Waters Corporation). Acquity UPLC BEH C18 (2.1 mm × 100 mm, 1.7 µm) was used as the column, 0.05% trifluoroacetic acid aqueous solution was used as the mobile phase A, and 0.05% trifluoroacetic acid acetonitrile solution was used as the mobile phase B. The gradient (%B) was 0/10, 4/90, 6/90, and 6.1/10, the flow rate was 0.3 ml/min, the column temperature was 40°C or less, and the diluents were acetonitrile and water. In addition, ELSD was used as a detector.

In addition, in the following Examples, X-select phenyl hexyl (250 mm × 19 mm, 5 µm) was used as a column for purification by a preparative HPLC method. In addition, a 0.1% formic acid aqueous solution was used as the mobile phase A, and an acetonitrile solution was used as the mobile phase B. The gradient (T/B%)was 0/40, 1/40, 11/45, 11.1/100, 13/100, 13.1/40, and 15/40, the flow rate was 19 ml/min, and the solvents were acetonitrile, water, and tetrahydrofuran.

Further, in the following Examples, combi flash-NEXTGEN 300 (manufactured by Teledyne Isco Inc.) was used for silica gel column chromatography.

### (Example 1)

The compound (vi) ((Z)-7-((1R,2R,3R,5S)-3,5-dihydroxy-2-((S,E)-3-hydroxy-8-methylnon-1-ene-1-yl)cyclopentyl)hepto-5-enoic acid) was manufactured by the following Reaction Scheme 4:

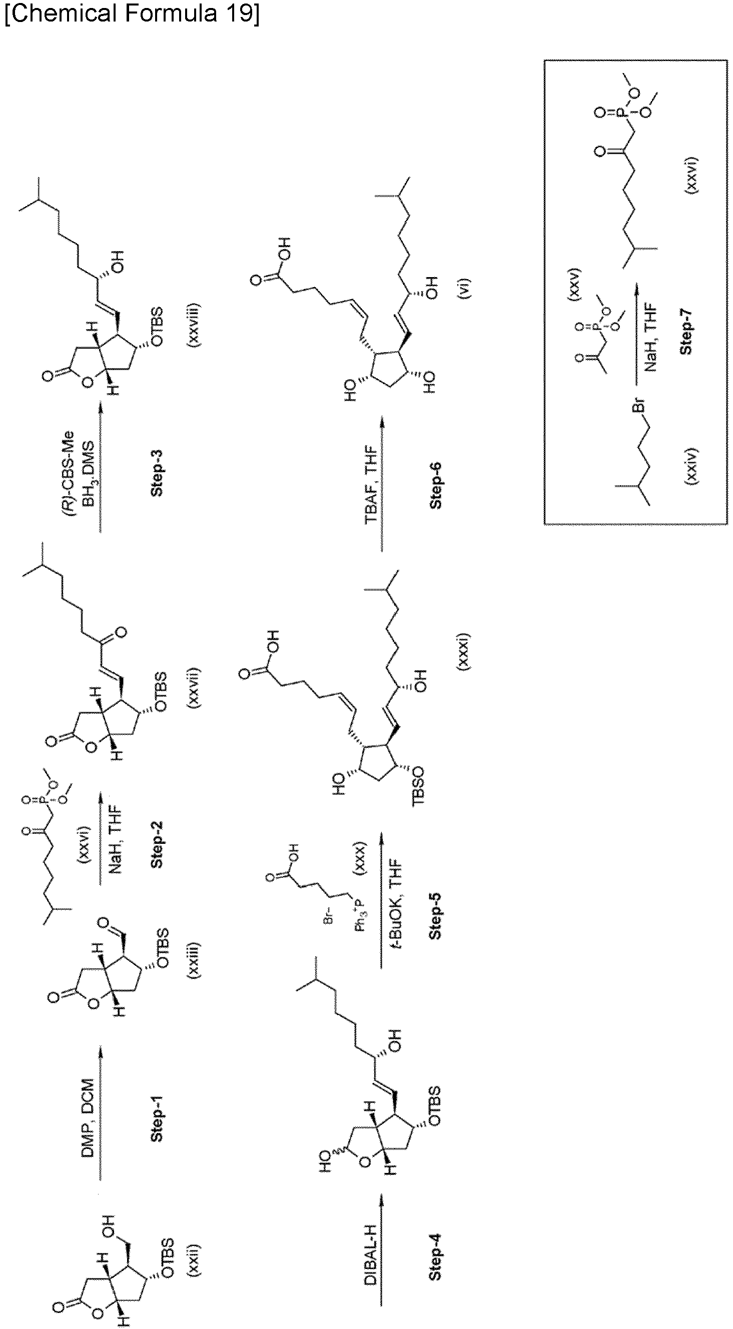

The Reaction Scheme 4 included the following steps of 1 to 7.

### (Step 1: Manufacturing of (3aR,4R,5R,6aS)-5-((tert-butyldimethylsilyl)oxy)-2-oxohexahydro-2H-cyclopenta[b]furan-4-carbaldehyde)

### Step 1:

A compound (xxiii) represented by a Chemical Formula (xxiii), ((3aR,4R,5R,6aS)-5-((tert-butyldimethylsilyl)oxy)-2-oxohexahydro-2H-cyclopenta[b]furan-4-carbaldehyde) was manufactured from a compound (xxii) represented by a Chemical Formula (xxii), ((3aR,4S,5R,6aS)-5-((tert-butyldimethylsilyl)oxy)-4-(hydroxymethyl)hexahydro-2H-cyclopenta[b]furan-2-one) in

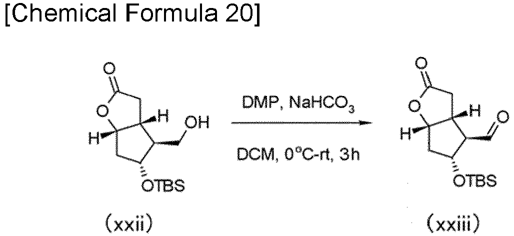

First, 5.00 g (17.45 mmol) of the compound (xxii) was dissolved in 45 ml of dichloromethane, and 9.50 g (34.0 mmol) of Dess-Martin periodinane (DMP) and 7.32 g (87.25 mmol) of sodium hydrogen carbonate were added thereto.

This mixture was stirred at 0°C to room temperature for 3 hours. The progress of the reaction was confirmed by TLC (Rf value of 0.6, 40% ethyl acetate/hexane). After completion of the reaction, the mixture was cooled with 100 ml of a saturated aqueous solution of sodium thiosulfate. After further stirring the mixture for 10 min, the mixture was diluted with 200 ml of dichloromethane and 100 ml of a saturated aqueous solution of ammonium chloride. Layers were separated and the aqueous layer was extracted twice with 100 ml of dichloromethane. The combined organic layer was washed with 100 ml of water and 100 ml of brine (salt water), dried over anhydrous sodium sulfate, then filtered and concentrated under reduced pressure to provide a crude reaction mixture.

The obtained crude reaction mixture was purified once by silica gel column chromatography using 10 to 30% by volume of ethyl acetate/hexane as an eluent to provide 4.50 g (15.8 mmol) of a white solid product.

From ¹H NMR spectrum, the obtained white solid was estimated to be the compound (xxiii).

### (Step 7: Manufacturing of dimethyl(7-methyl-2-oxooctyl)phosphonate)

### Step 7:

A compound (xxvi) represented by a Chemical Formula (xxvi), (dimethyl(7-methyl-2-oxooctyl)phosphonate) was manufactured from a compound (xxiv) represented by a Chemical Formula (xxiv), (1-bromo-4-methylpentane) and a compound (xxv) represented by a Chemical Formula (xxv), (dimethyl(2-oxopropyl)phosphonate) in

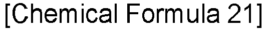

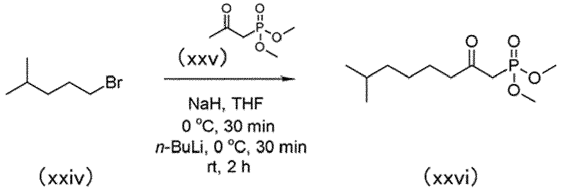

First, 20.0 g (120 mmol) of the compound (xxv) was dissolved in 240 ml of tetrahydrofuran, and 6.50 g (181 mmol, diluted to 60% in mineral oil) of sodium hydride was added thereto at 0°C. This mixture was stirred at 0°C for 30 minutes, and 73 ml of n-butyllithium (diluted to 2.5 M with tetrahydrofuran, 181 mmol) was added thereto.

This mixture was stirred at 0°C for 30 minutes, a solution obtained by dissolving 19.8 g (120 mmol) of the compound (xxiv) in 100 ml of tetrahydrofuran was added thereto, and was stirred at room temperature for 2 hours. The progress of the reaction was confirmed by TLC (Rf value of 0.3, 60% ethyl acetate/hexane). After completion of the reaction, the mixture was cooled with 50 ml of a saturated aqueous solution of ammonium chloride. Layers were separated and the aqueous layer was extracted three times with 200 ml of ethyl acetate. The combined organic layer was washed with 300 ml of water and 200 ml of brine (salt water), dried over anhydrous sodium sulfate, and then the solvent was removed under reduced pressure to provide a crude reaction mixture.

The obtained crude compound was purified once by silica gel column chromatography using 40 to 60% by volume of ethyl acetate/hexane as an eluent to provide 15.2 g (60.8 mmol) of a pale yellow liquid product.

From ¹H NMR spectrum, the obtained pale yellow liquid was estimated to be the compound (xxvi).

### (Step 2: Manufacturing of (3aR,4R,5R,6aS)-5-((tert-butyldimethylsilyl)oxy)-4-((E)-8-methyl-3-oxonone-1-ene-1-yl)hexahydro-2H-cyclopenta[b]furan-2-one)

### Step 2:

A compound (xxvii) represented by a Chemical Formula (xxvii), ((3aR,4R,5R,6aS)-5-((tert-butyldimethylsilyl)oxy)-4-((E)-8-methyl-3-oxonone-1-ene-1-yl)hexahydro-2H-cyclopenta[b]furan-2-one) was manufactured from the compound (xxiii) and compound (xxvi) in

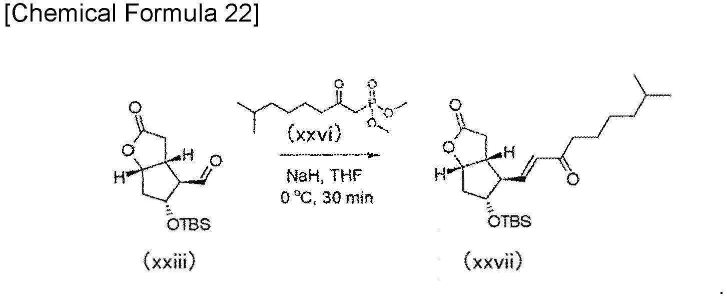

First, 1.90 g (47.5 mmol, diluted to 60% with mineral oil) of sodium hydride was suspended in 100 ml of tetrahydrofuran, and a solution obtained by dissolving 11.80 g (47.2 mmol) of the compound (xxvi) in 100 ml of tetrahydrofuran was added thereto at 0°C. The mixture was stirred at 0°C for 30 minutes, a solution obtained by dissolving 9.00 g (31.6 mmol) of the compound (xxiii) in 50 ml of tetrahydrofuran was added thereto, and the mixture was stirred at 0°C for 30 minutes. The progress of the reaction was confirmed by TLC (Rf value of 0.6, 30% ethyl acetate/hexane). After completion of the reaction, the mixture was cooled with 100 ml of a saturated aqueous solution of ammonium chloride.

Layers were separated and the aqueous layer was extracted three times with 200 ml of ethyl acetate. The combined organic layer was washed with 200 ml of water and 200 ml of brine (salt water), dried over anhydrous sodium sulfate, and then the solvent was removed under reduced pressure to provide a crude reaction mixture.

The obtained crude compound was purified once by silica gel column chromatography using 10 to 30% by volume of ethyl acetate/hexane as an eluent to provide 9.00 g (22.05 mmol) of a pale yellow liquid product.

From 1H NMR spectrum, the obtained pale yellow liquid was estimated to be the compound (xxvii).

### (Step 3: Manufacturing of (3aR,4R,5R,6aS)-5-((tert-butyldimethylsilyl)oxy)-4-((S,E)-3-hydroxy-8-methylnon-1-ene-1-yl)hexahydro-2H-cyclopenta[b]furan-2-one)

### Step 3:

A compound (xxviii) represented by a Chemical Formula (xxviii), ((3aR,4R,5R,6aS)-5-((tert-butyldimethylsilyl)oxy)-4-((S,E)-3-hydroxy-8-methylnon-1-ene-1-yl)hexahydro-2H-cyclopenta[b]furan-2-one) was manufactured from the compound (xxvii) in

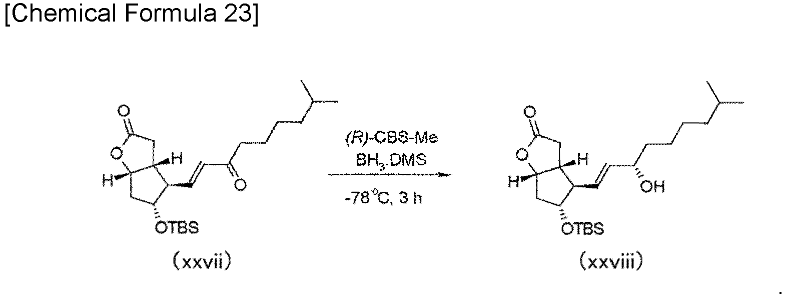

First, 8.00 g (29.4 mmol) of (R)-(-)-2-methyl CBS-oxazaboridine was dissolved in 70 ml of tetrahydrofuran, and 57 ml (88.2 mmol) of dimethylsulfide borane was added thereto at -78°C. This mixture was stirred at -78°C for 1 hour, a solution obtained by dissolving 6.00 g (14.7 mmol) of the compound (xxvii) in 80 ml of tetrahydrofuran was added thereto, and was stirred at -78°C for 2 hours. The progress of the reaction was confirmed by TLC (Rf value of 0.5, 40% ethyl acetate/hexane).

After completion of the reaction, the mixture was cooled with 100 ml of methanol and concentrated under reduced pressure. The crude residue was diluted with water and extracted three times with 150 ml of ethyl acetate. The combined organic layer was washed with 100 ml of water and 100 ml of brine (salt water), dried over anhydrous sodium sulfate and then concentrated under reduced pressure to provide a crude reaction mixture.

The obtained crude compound was purified once by silica gel column chromatography using 10 to 40% by volume of ethyl acetate/hexane as an eluent to provide 4.30 g (10.4 mmol) of a pale yellow liquid product.

From ¹H NMR spectrum, the obtained pale yellow liquid was estimated to be the compound (xxviii).

### (Step 4: Manufacturing of (3aR,4R,5R,6aS)-5-((tert-butyldimethylsilyl)oxy)-4-((S,E)-3-hydroxy-8-methylnon-1-ene-1-yl)hexahydro-2H-cyclopenta[b]furan-2-ol)

### Step 4:

A compound (xxix) represented by a Chemical Formula (xxix), ((3aR,4R,5R,6aS)-5-((tert-butyldimethylsilyl)oxy)-4-((S,E)-3-hydroxy -8-methylnon-1-ene-1-yl)hexahydro-2H-cyclopenta[b]furan-2-ol) was manufactured from the compound (xxviii) in

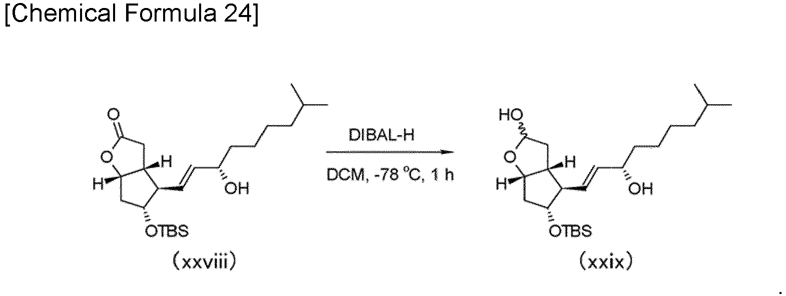

First, 4.30 g (10.4 mmol) of the compound (xxviii) was dissolved in 25 ml of dichloromethane, and 33.2 ml (33.2 mmol, diluted to 1 M with toluene) of diisobutylaluminum hydride was added thereto at -78°C. This mixture was stirred at -78°C for 1 hour. The progress of the reaction was confirmed by TLC (Rf value of 0.4, 40% ethyl acetate/hexane).

After completion of the reaction, the mixture was cooled with 40 ml of a saturated aqueous solution of potassium sodium tartrate. Layers were separated and the aqueous layer was extracted three times with 100 ml of dichloromethane. The combined organic layer was dried over anhydrous sodium sulfate and concentrated under reduced pressure to provide about 4.50 g of a yellow liquid product, which was used in the next reaction without further purification.

As a result of LCMS analysis, the obtained yellow liquid was estimated to be the compound (xxix).

### (Step 5: Manufacturing of (Z)-7-((1R,2R,3R,5S)-3-((tert-butyldimethylsilyl)oxy)-5-hydroxy-2-((S,E)-3-hydroxy-8-methylnon-1-ene-1-yl)cyclopentyl)hepto-5-enoic acid)

### Step 5:

A compound (xxxi) represented by a Chemical Formula (xxxi), ((Z)-7-((1R,2R,3R,5S)-3-((tert-butyldimethylsilyl)oxy)-5-hydroxy-2-((S,E)-3-hydroxy-8-methylnon-1-ene-1-yl)cyclopentyl)hepto-5-enoic acid) was manufactured from the compound (xxix) and a compound (xxx) represented by a Chemical Formula (xxx), ((4-carboxybutyl)triphenylphosphonium bromide) in

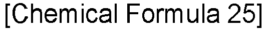

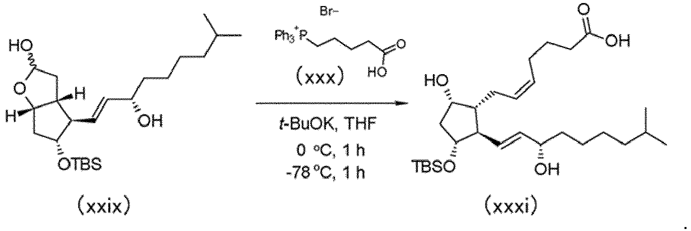

First, 24.2 g (54.6 mmol) of the compound (xxx) was dissolved in 70 ml of tetrahydrofuran, and 12.14 g (109 mmol) of potassium tert-butoxide was added thereto at 0°C. The mixture was stirred at 0°C for 1 hour, a solution of 4.5 g (10.9 mmol) of the compound (xxix) in 40 ml of anhydrous tetrahydrofuran was added thereto at -78°C.

The mixture was stirred at -78°C for 1 hour. The progress of the reaction was confirmed by TLC (Rf value of 0.5, 100% ethyl acetate). After completion of the reaction, the mixture was cooled with 100 ml of a saturated aqueous solution of ammonium chloride, acidified to pH 4 with 2 ml of 1 N hydrochloric acid and extracted twice with 250 ml of ethyl acetate. The combined organic layer was dried over sodium sulfate and concentrated under reduced pressure to provide a crude reaction mixture.

The obtained crude reaction mixture was purified once by silica gel column chromatography using 20 to 90% by volume of ethyl acetate/hexane as an eluent to provide 3.50 g (7.05 mmol) of a white solid product.

As a result of ¹H NMR and LCMS analysis, the obtained white solid was estimated to be the compound (xxxi).

### (Step 6: Manufacturing of (Z)-7-((1R,2R,3R,5S)-3,5-dihydroxy-2-((S, E)-3-hydroxy-8-methylnon-1-ene-1-yl)cyclopentyl) hepto-5-enoic acid)

### Step 6:

The compound (vi), ((Z)-7-((1R,2R,3R,5S)-3,5-dihydroxy-2-((S,E)-3-hydroxy-8-methylnon-1-ene-1-yl)cyclopentyl)hepto-5-enoic acid) was manufactured from the compound (xxxi) in

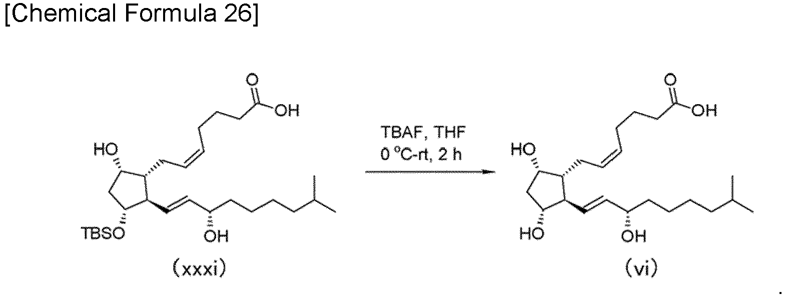

First, 3.50 g (7.05 mmol) of the compound (xxxi) was dissolved in 20 ml of tetrahydrofuran, and 8.0 ml (8 mmol, diluted to 1 M with tetrahydrofuran) of tetra-n-butylammonium fluoride was added thereto at 0°C. This mixture was stirred at room temperature for 2 hours. The progress of the reaction was confirmed by TLC (Rf value of 0.2, 100% ethyl acetate). After completion of the reaction, the mixture was concentrated under reduced pressure to provide a crude reaction mixture.

2.00 g of the obtained crude reaction mixture was purified by preparative HPLC to provide 560 mg (1.46 mmol) of a yellow solid product.

As a result of ¹H NMR and LCMS analysis, the obtained yellow solid was estimated to be the compound (vi).

### (Example 2)

The compound (vii) (isopropyl (Z)-7-((1R,2R,3R,5S)-3,5-dihydroxy-2-((S,E)-3-hydroxy-8-methylnon-1-ene-1-yl)cyclopentyl) hepto-5-enoate) was manufactured by the following Reaction Scheme 5:

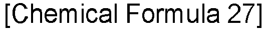

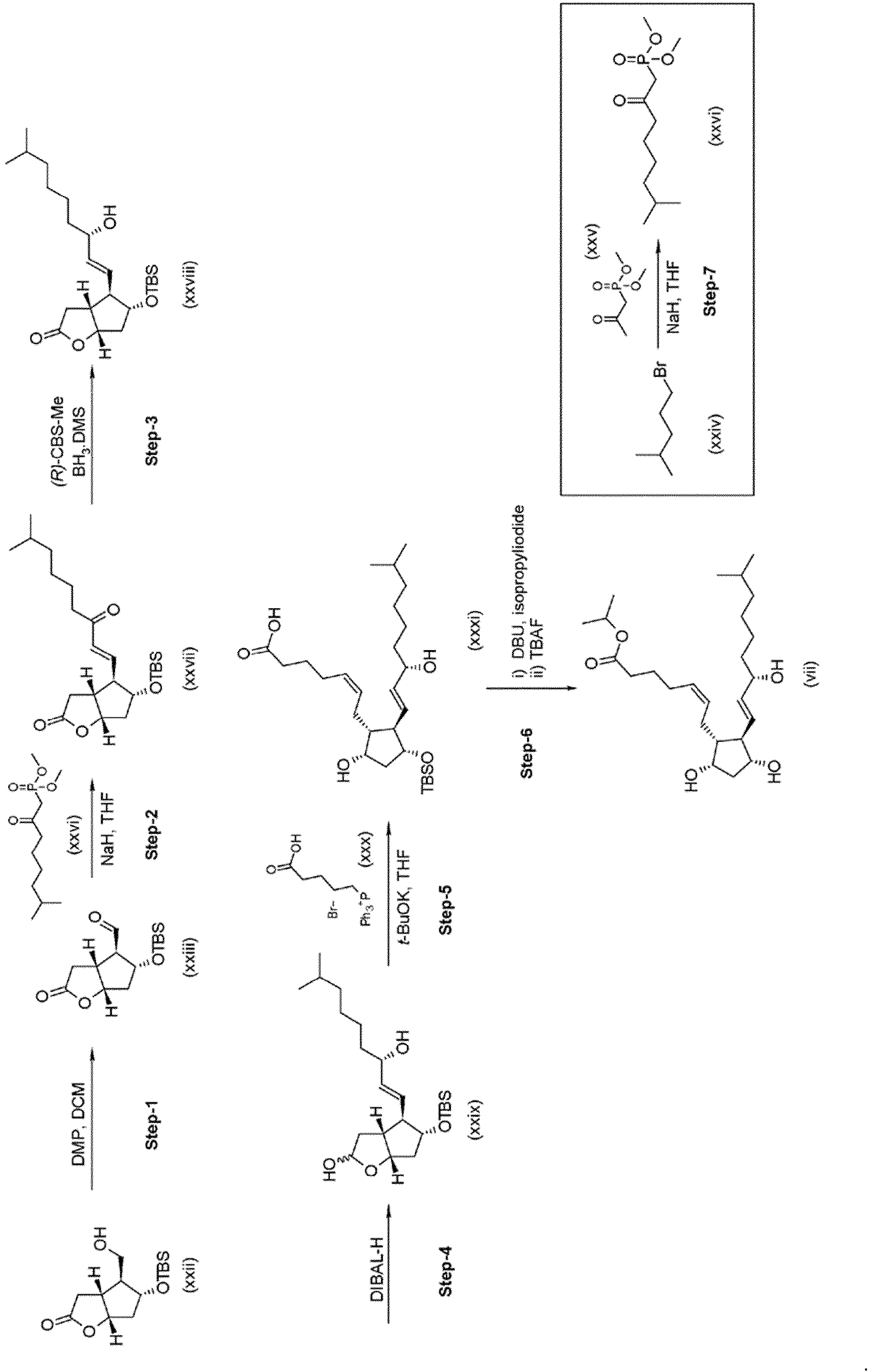

The processes of the steps 1 to 7 were included in the Reaction Scheme 5, but the steps 1 to 5 and 7 were the same processes as the steps 1 to 5 and 7 in Example 1.

Also in Example 2, the compound (xxxi) was manufactured through the same steps as the steps 1 to 5 and 7 in Example 1.

### (Step 6: Manufacturing of (isopropyl (Z)-7-((1R,2R,3R,5S)-3,5-dihydroxy-2-((S,E)-3-hydroxy-8-methylnon-1-ene-1-yl)cyclopentyl)hepto-5-enoate)

### Step 6:

The compound (vii), (isopropyl (Z)-7-((1R,2R,3R,5S)-3,5-dihydroxy-2-((S,E)-3-hydroxy-8-methylnon-1-ene-1-yl)cyclopentyl)hepto-5-enoate) was manufactured from the compound (xxxi) in

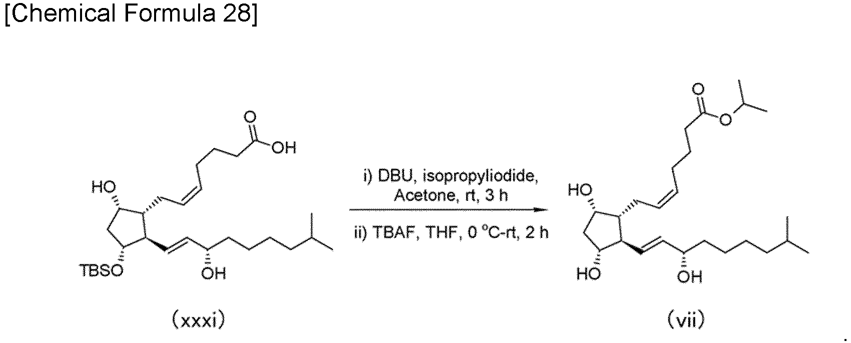

First, 2.00 g (4.02 mmol) of the compound (xxxi) was dissolved in 10 ml of acetonitrile, and a solution obtained by dissolving 9.0 ml (20 mmol) of 1,8-diazobicyclo[5.4.0]undec-7-ene (DBU) in 10 ml of acetonitrile was added thereto.

This mixture was stirred at room temperature for 10 minutes, and a solution obtained by dissolving 5.0 ml (12 mmol) of 2-iodopropane in 10 ml of acetonitrile was added. The mixture was stirred at room temperature for 3 hours. After completion of the reaction, the mixture was concentrated under reduced pressure and diluted with 200 ml of ethyl acetate. The organic layer was washed twice with 20 ml of a 3% aqueous citric acid solution, twice with 20 ml of a 5% aqueous sodium hydrogen carbonate solution, once with 50 ml of brine (salt water), dried over anhydrous sodium sulfate, and then concentrated under reduced pressure to provide a crude reaction mixture.

The obtained crude reaction mixture was purified once by silica gel column chromatography using 20 to 40% by volume of ethyl acetate/hexane as an eluent to provide 2.00 g of a pale yellow liquid.

The resulting pale yellow liquid of 2.00 g was dissolved in 20 ml of tetrahydrofuran, and 8.0 ml (8 mmol, diluted to 1 M with tetrahydrofuran) of tetra-n-butylammonium fluoride was added thereto at 0°C. The mixture was stirred at room temperature for 2 hours. The progress of the reaction was confirmed by TLC (Rf value of 0.6, 50% ethyl acetate/hexane). After completion of the reaction, the reaction mixture was concentrated under reduced pressure to provide a crude reaction mixture.

The obtained crude reaction mixture of 1.85 g was purified by preparative HPLC to provide 400 mg (0.94 mmol) of a pale yellow liquid.

As a result of ¹H NMR and LCMS analysis, the obtained pale yellow liquid was estimated to be the compound (vii).

### [Preparation and evaluation of compositions]

### (Preparation of solution A)

87.736889990 parts by mass of water, 5.0030 parts by mass of butylene glycol, 2.0 parts by mass of glycerin, 1.0 part by mass of pyrrolidinyl diaminopyrimidine oxide (cosmetic minoxidil-like component), 1.0 part by mass of 3-O-ethylascorbic acid, 0.970 parts by mass of human stem cell adapted culture solution, 0.930 parts by mass of 1,2-hexanediol, 0.50 parts by mass of acetylcysteine, 0.20 parts by mass of phenoxyethanol, 0.150 parts by mass of methylparaben, 0.10 parts by mass of riboflavin, 0.10 parts by mass of pyridoxine hydrochloride, 0.10 parts by mass of hydroxyethyl cellulose, 0.10 parts by mass of sodium hyaluronate, 0.10 parts by mass of nicotinamide adenine dinucleotide, 0.010 parts by mass of tocopheryl sodium phosphate, 0.00010 parts by mass of human gene recombinant oligopeptide-1 (EGF), 0.000010 parts by mass of human gene recombinant polypeptide-7 (FGF), and 0.000000010 parts by mass of synthetic human gene recombinant polypeptide-31 (KGF) were mixed to provide a solution A. The components and contents of the solution A are shown in Table 1.

### (Preparation of solution B)

89.73990 parts by mass of water, 5.0 parts by mass of butylene glycol, 2.0 parts by mass of glycerin, 1.0 part by mass of 3-O-ethyl ascorbic acid, 0.90 parts by mass of 1,2-hexanediol, 0.50 parts by mass of acetylcysteine, 0.20 parts by mass of phenoxyethanol, 0.150 parts by mass of methylparaben, 0.10 parts by mass of riboflavin, 0.10 parts by mass of pyridoxine hydrochloride, 0.10 parts by mass of hydroxyethyl cellulose, 0.10 parts by mass of sodium hyaluronate, 0.10 parts by mass of nicotinamide adenine dinucleotide, 0.010 parts by mass of tocopheryl sodium phosphate, and 0.00010 parts by mass of human gene recombinant oligopeptide-1 (EGF) were mixed to provide a solution B. The components and contents of the solution B are shown in Table 1.

**[Table 1]**

| Component | Solution A (parts by mass) | Solution B (parts by mass) |
|---|---|---|
| Water | 87.736889990 | 89.73990 |
| Butylene glycol | 5.0030 | 50 |
| Glycerin | 2.0 | 20 |
| Pyrrolidinyl diaminopyrimidine oxide (cosmetic minoxidyl-like component) | 1.0 | - |
| 3-O-Ethylascorbic acid | 1.0 | 1.0 |
| Human stem cell adapted culture solution | 0.970 | - |
| 1,2-Hexanediol | 0.930 | 0.90 |
| Acetylcysteine | 0.50 | 0.50 |
| Phenoxyethanol | 0.20 | 0.20 |
| Methylparaben | 0.150 | 0.150 |
| Riboflavin | 0.10 | 0.10 |
| Pyridoxine hydrochloride | 0.10 | 0.10 |
| Hydroxyethyl cellulose | 0.10 | 0.10 |
| Sodium hyaluronate | 0.10 | 0.10 |
| Nicotinamide adenine dinucleotide | 0.10 | 0.10 |
| Tocopheryl sodium phosphate | 0.010 | 0.010 |
| Human gene recombinant oligopeptide-1 (EGF) | 0.00010 | 0.00010 |
| Human gene recombinant polypeptide-7 (FGF) | 0.000010 | - |
| Synthetic human gene recombinant polypeptide-31 (KGF) | 0.000000010 | - |
| Total | 100 | 100 |

### (Evaluation of growth effect on eyelashes of composition)

The eyelash growth effect was evaluated by instilling the composition into the eyeballs of rabbits and evaluating the extension of the length of the eyelashes for 21 days. The instillation test was performed in Examples 3 to 8 and Comparative Examples 1 and 2 from May 20, 2021 to June 10, 2021, and in Reference Examples 1 to 3 from September 2, 2021 to September 22, 2021.

For each composition, an instillation test was performed on five rabbits. That is, the number of rabbits subjected to the instillation test was 11 groups for each of 5 rabbits, and 55 rabbits in total. Grouping was performed randomly so that the weight of the rabbit was not biased among the groups.

The sexes of the rabbits subjected to the instillation test were all male. During the instillation test, rabbits were kept individually in stainless steel cages. The ventilation in the breeding room was performed 10 to 15 times per 1 hour, the temperature during breeding was 19.5 to 22.6°C, and the humidity was 48 to 65%. In addition, the lighting was turned on for 12 hours and turned off for 12 hours in one day. Rabbits were fed a commercial feed and RO water.

Rabbits were instilled twice daily in the morning and evening with the composition. The instillation was applied to the left eye of a rabbit, and the amount of the instillation was 50 µl each time.

The instillation was performed so that the composition entered the conjunctival sac of the left eye after the lower eyelid of the rabbit was gently pulled away from the eyeball. The eyelids were then lightly pressed for about 30 seconds to prevent loss of the composition. After the instillation, the rabbits were returned to their respective cages.

The measurement of the length of the eyelashes was performed before the start of the instillation test (day 0) and on the 2nd, 7th, 14th, and 21st days after the start of the instillation test. When measuring the eyelashes, the largest center eyelash among the eyelashes of the lower eyelid of the rabbit was measured with a graduated measure. The difference between the length of the eyelashes on each measurement day and the length of the eyelashes before the start of the instillation test (day 0) was taken as the extension of the length of the eyelashes.

### (Example 3)

The compound (vi) of 0.03 parts by mass was added to physiological saline, and was dissolved by stirring to provide a composition 1.

The mean eyelash length of rabbits in the group to which the composition 1 was instilled (hereinafter, referred to as group 1) was 1.08 cm on day 0, 1.10 cm on the 2nd day, 1.18 cm on the 7th day, 1.22 cm on the 14th day, and 1.26 cm on the 21st day. That is, the eyelashes of the rabbits in the group 1 extended by 0.02 cm for 2 days, 0.10 cm for 7 days, 0.14 cm for 14 days, and 0.18 cm for 21 days on average. The results of evaluating the growth effect on eyelashes are shown in Table 2.

### (Example 4)

The compound (vii) of 0.03 parts by mass was added to physiological saline, and was dissolved by stirring to provide a composition 2.

The mean eyelash length of rabbits in the group to which the composition 2 was instilled (hereinafter, referred to as group 2) was 1.02 cm on day 0, 1.06 cm on the 2nd day, 1.14 cm on the 7th day, 1.18 cm on the 14th day, and 1.24 cm on the 21st day. That is, the eyelashes of the rabbits in the group 2 extended by 0.04 cm for 2 days, 0.12 cm for 7 days, 0.16 cm for 14 days, and 0.22 cm for 21 days on average. The results of evaluating the growth effect on eyelashes are shown in Table 2.

### (Comparative Example 1)

The average length of eyelashes of rabbits in the group (hereinafter, referred to as group 3) to which physiological saline (hereinafter, referred to as composition 3) was instilled was 1.00 cm on day 0, 1.04 cm on 2 days, 1.06 cm on 7 days, 1.14 cm on 14 days, and 1.16 cm on 21 days. That is, the eyelashes of the rabbits in the group 3 extended by 0.04 cm for 2 days, 0.06 cm for 7 days, 0.14 cm for 14 days, and 0.16 cm for 21 days on average. The results of evaluating the growth effect on eyelashes are shown in Table 2.

### (Example 5)

The compound (vi) of 0.03 parts by mass was added to the solution B and dissolved by stirring to provide a composition 4.

The mean eyelash length of rabbits in the group to which the composition 4 was instilled (hereinafter, referred to as group 4) was 1.04 cm on day 0, 1.08 cm on the 2nd day, 1.14 cm on the 7th day, 1.20 cm on the 14th day, and 1.26 cm on the 21st day. That is, the eyelashes of the rabbits in the group 4 extended by 0.04 cm for 2 days, 0.10 cm for 7 days, 0.16 cm for 14 days, and 0.22 cm for 21 days on average. The results of evaluating the growth effect on eyelashes are shown in Table 2.

### (Example 6)

The compound (vii) of 0.03 parts by mass was added to the solution B and dissolved by stirring to provide a composition 5.

The mean eyelash length of rabbits in the group to which the composition 5 was instilled (hereinafter, referred to as group 5) was 0.96 cm on day 0, 0.98 cm on the 2nd day, 1.06 cm on the 7th day, 1.12 cm on the 14th day, and 1.16 cm on the 21st day. That is, the eyelashes of the rabbits in the group 5 extended by 0.02 cm for 2 days, 0.10 cm for 7 days, 0.16 cm for 14 days, and 0.20 cm for 21 days on average. The results of evaluating the growth effect on eyelashes are shown in Table 2.

### (Comparative Example 2)

The average length of eyelashes of rabbits in the group (hereinafter, referred to as group 6) to which the solution A (hereinafter, referred to as composition 6) was instilled was 0.98 cm on day 0, 1.00 cm on the 2nd day, 1.02 cm on the 7th day, 1.10 cm on the 14th day, and 1.12 cm on the 21st day. That is, the eyelashes of the rabbits in the group 6 extended by 0.02 cm for 2 days, 0.04 cm for 7 days, 0.12 cm for 14 days, and 0.14 cm for 21 days on average. The results of evaluating the growth effect on eyelashes are shown in Table 2.

### (Example 7)

The compound (vi) of 0.03 parts by mass was added to the solution A and dissolved by stirring to provide a composition 7.

The mean eyelash length of rabbits in the group to which the composition 7 was instilled (hereinafter, referred to as group 7) was 1.04 cm on day 0, 1.08 cm on the 2nd day, 1.12 cm on the 7th day, 1.18 cm on the 14th day, and 1.26 cm on the 21st day. That is, the eyelashes of the rabbits in the group 7 extended by 0.04 cm for 2 days, 0.08 cm for 7 days, 0.14 cm for 14 days, and 0.22 cm for 21 days on average. The results of evaluating the growth effect on eyelashes are shown in Table 2.

### (Example 8)

The compound (vii) of 0.03 parts by mass was added to the solution A and dissolved by stirring to provide a composition 8.

The mean eyelash length of rabbits in the group to which the composition 8 was instilled (hereinafter, referred to as group 8) was 1.00 cm on day 0, 1.00 cm on the 2nd day, 1.04 cm on the 7th day, 1.12 cm on the 14th day, and 1.16 cm on the 21st day. That is, the eyelashes of the rabbits in the group 8 extended by 0 cm for 2 days, 0.04 cm for 7 days, 0.12 cm for 14 days, and 0.16 cm for 21 days on average. The results of evaluating the growth effect on eyelashes are shown in Table 2.

### (Reference Example 1)

The average length of eyelashes of rabbits in the group (hereinafter, referred to as group 9) to which the solution B (hereinafter, referred to as composition 9) was instilled was 0.892 cm on day 0, 0.910 cm on the 2nd day, 0.916 cm on the 7th day, 0.924 cm on the 14th day, and 0.928 cm on the 21st day. That is, the eyelashes of the rabbits in the group 9 extended by 0.018 cm for 2 days, 0.024 cm for 7 days, 0.032 cm for 14 days, and 0.036 cm for 21 days on average. The results of evaluating the growth effect on eyelashes are shown in Table 2.

### (Reference Example 2)

The mean eyelash length of rabbits in the group (hereinafter, referred to as group 10) to which LUMIGAN (0.03% bimatoprost manufactured by Allergan Inc., hereinafter referred to as composition 10) was instilled was 0.826 cm on day 0, 0.834 cm on the 2nd day, 0.854 cm on the 7th day, 0.862 cm on the 14th day, and 0.872 cm on the 21st day. That is, the eyelashes of the rabbits in the group 10 extended by 0.008 cm for 2 days, 0.028 cm for 7 days, 0.036 cm for 14 days, and 0.046 cm for 21 days on average. The results of evaluating the growth effect on eyelashes are shown in Table 2.

### (Reference Example 3)

The average length of eyelashes of rabbits in the group (hereinafter, referred to as group 11) to which physiological saline (hereinafter, referred to as composition 11) was instilled was 0.810 cm on day 0, 0.818 cm on the 2nd day, 0.824 cm on the 7th day, 0.818 cm on the 14th day, and 0.846 cm on the 21st day. That is, the eyelashes of the rabbits in the group 11 extended by 0.008 cm for 2 days, 0.014 cm for 7 days, 0.008 cm for 14 days, and 0.036 cm for 21 days on average. The results of evaluating the growth effect on eyelashes are shown in Table 2.

**[Table 2]**

| | Composition | | Mean eyelash length (cm) | | | | | Mean eyelash extension (cm) | | | |
|---|---|---|---|---|---|---|---|---|---|---|---|
| | | | 0 day | 2nd day | 7th day | 14th day | 21st day | 2 days | 7 days | 14 days | 21 days |
| Example 3 | 1 | Physiological saline, compound (vi) | 1.08 | 1.10 | 1.18 | 1.22 | 1.26 | 0.02 | 0.10 | 0.14 | 0.18 |
| Example 4 | 2 | Physiological saline, compound (vii) | 1.02 | 1.06 | 1.14 | 1.18 | 1.24 | 0.04 | 0.12 | 0.16 | 0.22 |
| Comparative Example 1 | 3 | Physiological saline | 1.00 | 1.04 | 1.06 | 1.14 | 1.16 | 0.04 | 0.06 | 0.14 | 0.16 |
| Example 5 | 4 | Solution B, compound (vi) | 1.04 | 1.08 | 1.14 | 1.20 | 1.26 | 0.04 | 0.10 | 0.16 | 0.22 |
| Example 6 | 5 | Solution B, compound (vii) | 0.96 | 0.98 | 1.06 | 1.12 | 1.16 | 0.02 | 0.10 | 0.16 | 0.20 |
| Comparative Example 2 | 6 | Solution A | 0.98 | 1.00 | 1.02 | 1.10 | 1.12 | 0.02 | 0.04 | 0.12 | 0.14 |
| Example 7 | 7 | Solution A, compound (vi) | 1.04 | 1.08 | 1.12 | 1.18 | 1.26 | 0.04 | 0.08 | 0.14 | 0.22 |
| Example 8 | 8 | Solution A, compound (vii) | 1.00 | 1.00 | 1.04 | 1.12 | 1.16 | 0 | 0.04 | 0.12 | 0.16 |
| Reference Example 1 | 9 | Solution B | 0.892 | 0.910 | 0.916 | 0.924 | 0.928 | 0.018 | 0.024 | 0.032 | 0.036 |
| Reference Example 2 | 10 | LUMIGAN (bimatoprost) | 0.826 | 0.834 | 0.854 | 0.862 | 0.872 | 0.008 | 0.028 | 0.036 | 0.046 |
| Reference Example 3 | 11 | Physiological saline | 0.810 | 0.818 | 0.824 | 0.818 | 0.846 | 0.008 | 0.014 | 0.008 | 0.036 |

From the results of evaluation of the growth effect on the eyelashes of Examples 3 and 4 and Comparative Example 1, it was confirmed that the average value of the extension of the eyelashes for 21 days of rabbits to which the composition containing the compound (vi) or (vii) of the present invention was instilled was larger than the average value of the extension of the eyelashes for 21 days of rabbits to which physiological saline was instilled. That is, it was considered that the compound (vi) or (vii) of the present invention promoted the extension of eyelashes of rabbits.

In addition, in Example 4 and Comparative Example 1, when a t-test was performed on the values of eyelash extension of 5 rabbits for 21 days, the one-side p value was 0.047. That is, it was confirmed that the composition containing the compound (vii) of the present invention significantly promoted the extension of the eyelashes of rabbits as compared with physiological saline.

It is known that the pyrrolidinyl diaminopyrimidine oxide (cosmetic minoxidil-like component) contained in the composition of Comparative Example 2 has an effect of promoting hair growth. From the results of evaluating the growth effect of eyelashes of Examples 5 and 6 and Comparative Example 2, it was confirmed that the composition containing the compound (vi) or (vii) of the present invention had a higher effect of promoting the extension of the eyelashes of rabbits than the composition containing pyrrolidinyl diaminopyrimidine oxide (cosmetic minoxidil-like component).

In Examples 3 to 8, Comparative Examples 1 and 2, and Reference Examples 1 and 3, no inflammation or pigmentation was observed in the rabbits' eyes.

In Reference Example 2, it was confirmed that slight inflammation occurred in the rabbits' eyes after instillation of the composition 10. Inflammation subsided in about 5 to 10 minutes after instillation of the composition 10. Bimatoprost contained in the composition 10 has an effect of promoting hair growth, but is known to have a side effect causing inflammation.

## Claims

1. A compound represented by Chemical Formula (i): 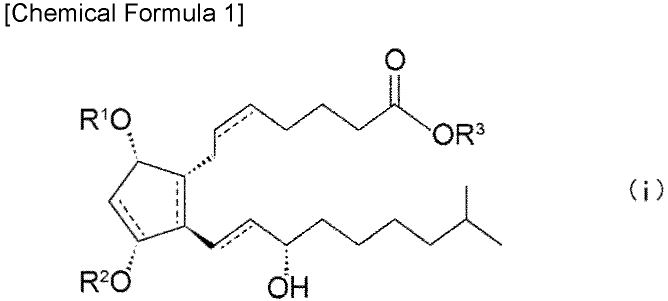 wherein
a bond indicated by parallel broken and solid lines represents a single bond or a double bond capable of taking a cis configuration or a trans configuration,
R¹ and R² each represent hydrogen, an alkyl group having 1 to 6 carbon atoms, a cycloalkyl group having 3 to 8 carbon atoms, an aryl group having 6 to 8 carbon atoms, or R⁴-C(=O)-, and R⁴ represents an alkyl group having 1 to 6 carbon atoms,
R³ represents hydrogen, an alkyl group having 1 to 6 carbon atoms, a cycloalkyl group having 3 to 8 carbon atoms, or an aryl group having 6 to 8 carbon atoms.

2. The compound according to claim 1, represented by Chemical Formula (vi): 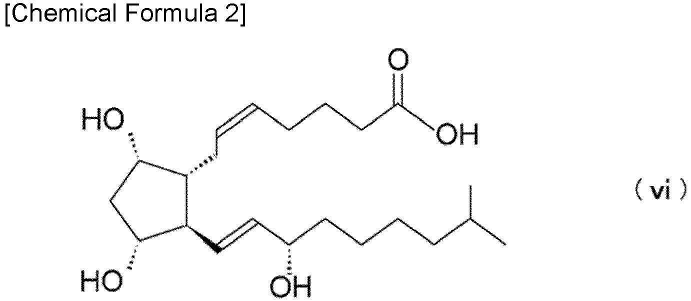 or Chemical Formula (vii):

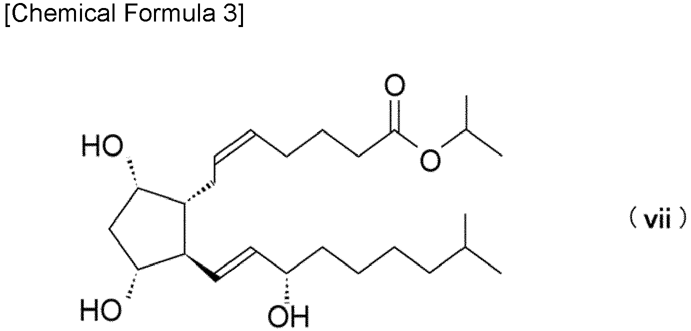

3. The compound according to claim 1 or 2, wherein the compound promotes hair growth of mammals.

4. A composition comprising the compound according to any one of claims 1 to 3 or a salt thereof.

5. The composition according to claim 4, wherein a content of the compound or a salt thereof is 0.0005 to 5.0% by mass.

6. The composition according to claim 4 or 5, comprising vitamins,
wherein
a content of the vitamins is 0.01 to 6.0% by mass.

7. A method for applying the composition according to any one of claims 4 to 6 to mammalian skin.

8. A manufacturing method for manufacturing a compound represented by Chemical Formula (ii): 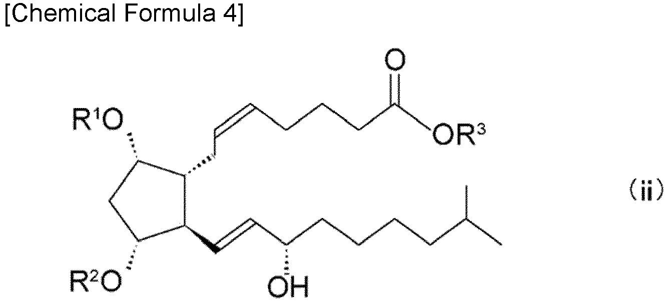 wherein
R¹ and R² each represent hydrogen, an alkyl group having 1 to 6 carbon atoms, a cycloalkyl group having 3 to 8 carbon atoms, an aryl group having 6 to 8 carbon atoms, or R⁴-C(=O)-, and R⁴ represents an alkyl group having 1 to 6 carbon atoms,
R³ represents hydrogen, an alkyl group having 1 to 6 carbon atoms, a cycloalkyl group having 3 to 8 carbon atoms, or an aryl group having 6 to 8 carbon atoms,
the manufacturing method comprising a step of condensation of a compound represented by Chemical Formula (ix): 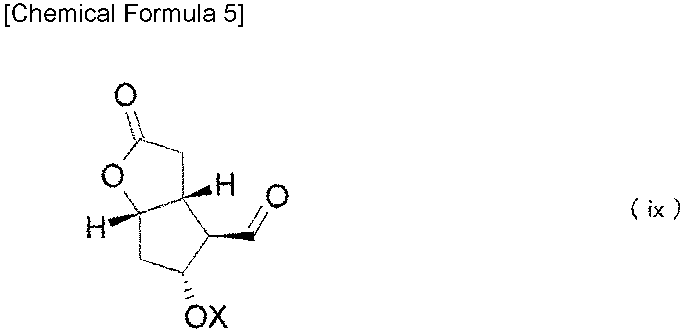 wherein
X represents a hydroxyl protecting group
and a compound represented by Chemical Formula (x): 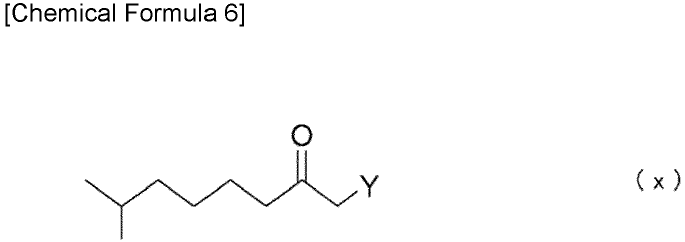 wherein
Y represents a leaving group
to manufacture a compound represented by Chemical Formula (xi): 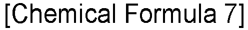 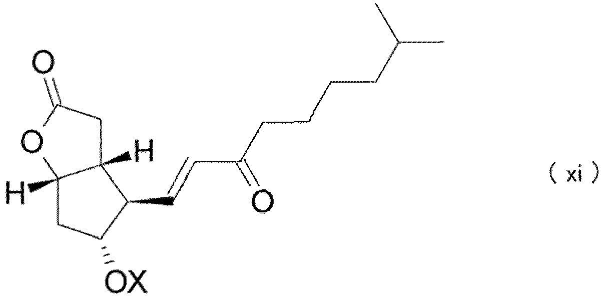 wherein
X represents a hydroxyl protecting group.
